# EUROPEAN PATENT SPECIFICATION

(11) **EP 3 810 279 B1**
(45) Date of publication and mention of the grant of the patent: **13.09.2023**
(21) Application number: 19734322.1
(22) Date of filing: 19.06.2019
(51) Int. Cl.: A61P 35/00, A61K 38/16, A61K 39/395

(54) **A COMBINATION COMPRISING USING AN OLIGOPEPTIDIC COMPOUND AND ANTI-PD-1 OR PD-L1 ANTIBODY FOR USE IN TREATING NEOPLASTIC CONDITIONS**
EINE KOMBINATION BESTEHEND AUS EINER OLIGOPEPTIDISCHEN VERBINDUNG UND EINEM ANTI-PD-1- ODER PD-L1-ANTIKÖRPER ZUR VERWENDUNG IN DER BEHANDLUNG VON NEOPLASTISCHEN STÖRUNGEN
UNE COMBINAISON D'UN COMPOSÉ OLIGOPEPTIDIQUE ET D'UN ANTICORPS ANTI-PD-1 OU PD-L1 POUR L'UTILISATION DANS LE TRAITEMENT D'AFFECTIONS NEOPLASIQUES

(30) Priority: 19.06.2018 GB 201810058
(43) Date of publication of application: 28.04.2021
(73) Proprietor: Cytovation ASA, 5058 Bergen (NO)
(72) Inventor: PRESTEGARDEN, Lars, 5058 Bergen (NO)
(74) Representative: Dehns
(86) International application number: PCT/EP2019/066295
(87) International publication number: WO 2019/243471

(56) References cited:
- WO-A1-2016/207646
- US-A1- 2015 038 407
- SUZANNE?L. TOPALIAN ET AL: "Immune Checkpoint Blockade: A Common Denominator Approach to Cancer Therapy", CANCER CELL, vol. 27, no. 4, 1 April 2015 (2015-04-01), pages 450-461, XP055372181, US ISSN: 1535-6108, DOI: 10.1016/j.ccell.2015.03.001

## Description

The present invention relates to a combination of an oligopeptidic compound with a checkpoint inhibitor for use in treating a neoplastic condition. In particular, the present invention provides an oligopeptidic compound which is selectively toxic to cancer cells (and thus has an anti-cancer effect) for use in combination with a checkpoint inhibitor for the treatment of cancer. Kits and products for use in treating a neoplastic condition comprising such an oligopeptidic compound and a checkpoint inhibitor are also provided.

Neoplastic conditions are medical conditions characterised by abnormal cell growth. Characteristically, the abnormal cell growth associated with neoplastic conditions results in the formation of a tumour (a solid mass of cells formed due to abnormal cell growth), though this is not always the case (particularly in neoplastic conditions of the blood). Neoplastic conditions may be malignant or benign. A benign tumour is unable to invade neighbouring tissues or to metastasise (i.e. to spread to other locations within the body of the patient in which it is present). However, a malignant tumour is able to do both of these things. Commonly, malignant tumours are known as cancers.

In 2010, across the world more people (about 8 million) died from cancer than any other single cause (Lozano et al., Lancet 380: 2095-2128, 2012). Moreover, as populations across the world age, cancer rates are expected to increase. There is thus an urgent need for new and improved therapies for cancer.

WO 2011/092347 discloses oligopeptidic compounds which are selectively cytotoxic for neoplastic cells. These oligopeptidic compounds include peptides consisting of the amino acid sequence set forth in SEQ ID NO: 1 (named CyPep-1). As detailed therein, and shown in the Examples below, peptides based on CyPep-1 hold great potential as a new therapeutic for cancer. CyPep-1 has not only been shown to be selectively cytotoxic towards cancer cells *in vitro,* it has also been shown to have a strong anti-tumour effect and to be well tolerated in animal models of disease.

CyPep-1 is a fusion peptide based on a fragment of the tumour suppressor protein Conductin/Axin2 coupled to the C-terminus of the HIV-TAT cell-penetrating peptide. The HIV-TAT cell-penetrating peptide is a cationic peptide, and without being bound by theory, it is believed that the selective cytotoxicity of CyPep-1 is due to the negative charge held by cancer cell membranes (in contrast, non-cancerous mammalian cells tend to have membranes with a more neutral charge). Beneficially, CyPep-1 also has antibacterial properties (possibly also due to the negative charge held by many bacterial cell membranes), and has been shown to have a potent bacteriocidal effect against medically-relevant species of Gram-positive and Gram-negative bacteria (see WO 2011/092347).

Immune checkpoint inhibitors (hereafter simply "checkpoint inhibitors") are a relatively new family of anti-cancer drugs which function by activating a patient's immune system to attack cancer cells. Checkpoint inhibitors act by blocking the activity of immune checkpoints. Immune checkpoints keep the immune system in check by preventing the killing of healthy cells and autoimmunity. They act as a "brake" on the immune system by preventing T-cell activation. Checkpoint proteins are expressed on the surface of immune cells and bind to checkpoint ligands on the surface of target cells or antigen-presenting cells, resulting in inhibition of immune cell activity.

The best known example of an immune checkpoint is PD-1 (programmed cell death protein 1), which is expressed by T-cells and binds PD-L1 (programmed death ligand 1) and PD-L2 expressed on the surface of cells including target cells, lymphocytes and antigen-presenting cells. Activation of PD-1 by PD-L1 or PD-L2 binding inhibits T-cell activation and proliferation. Up-regulation of PD-L1 and/or PD-L2 by cancer cells thus acts as a protective mechanism to prevent their destruction by T-cells. Up-regulation of PD-L1 and/or PD-L2 by healthy cells in the vicinity of a tumour has a similar dampening effect on the immune response. Another important immune checkpoint is CTLA-4 (cytotoxic T-lymphocyte antigen-4), which is also expressed on the surface of T-cells (primarily CD4+ T-cells). CTLA-4 binds CD80 and CD86 on the surface of antigen-presenting cells. CD80 and CD86 are also ligands for the T-cell co-stimulatory receptor CD28. CTLA-4 has a much higher affinity for CD80 and CD86 than does CD28, meaning that high expression of CTLA-4 by a T-cell leads to out-competing of CD28 for CD80/CD86 binding and thus the down-regulation of T-cell activity by inhibition of co-stimulation. Checkpoint inhibitors act by preventing (generally blocking) the interaction between an immune checkpoint and its ligand, thus up-regulating immune cell activity. Immune checkpoints, and their blockade in cancer therapy, are reviewed in Topalian et al., Cancer Cell 27: 450-461, 2015.

Despite their strong promise, the results of clinical trials of checkpoint inhibitors in cancer therapy have been mixed. While notable successes have occurred, responses to checkpoint inhibitors are generally seen in only a relatively small proportion of patients or in patients with only very specific types of cancers. A number of checkpoint inhibitors have been subject to trials, both as monotherapies and within combination therapies utilising a checkpoint inhibitor and a second anti-cancer therapeutic. While some of these therapies have been found highly effective (see e.g. Robert et al., N Engl J Med 372: 2521-2532, 2015, which reports the success of the anti-PD-1 antibody pembrolizumab in melanoma treatment and Liu et al., Nature Communications 8: 14754, 2017, which discloses the successful combination of a checkpoint inhibitor with an oncolytic virus in an animal model), many trials have failed (e.g. a trial of the combination of the small molecule epacadostat (Incyte, US) with pembrolizumab for melanoma treatment was halted after failing to improve progression-free survival in subjects, see the press release issued by Incyte and MSD on the subject on 6 April 2018). Whether a combination of a checkpoint inhibitor and a particular second therapeutic agent will be successful is unpredictable.

As detailed herein, the present inventor has surprisingly found that combination therapy using CyPep-1 and a checkpoint inhibitor (specifically an anti-PD1 antibody) to treat cancer results in a beneficial effect. In particular, we have shown that the efficacy of a checkpoint inhibitor can be enhanced by using the combination. Furthermore, the efficacy of CyPep-1 may be enhanced by using it in combination with a checkpoint inhibitor. For example, lower doses of CyPep-1 may be used than in monotherapy with CyPep-1. Thus, the combination of a CyPep-1 peptide in combination with a checkpoint inhibitor results in an enhanced therapeutic effect as compared with either the peptide or the checkpoint inhibitor alone. In particular, and in certain embodiments, it is believed that a synergistic effect is occurring, and that the data support that the combination results in a synergistic enhancement of the effect of the two drugs. Combining checkpoint inhibitor therapy (particularly anti-PD-1/PD-L1 therapy) with CyPep-1 therapy has been found to enhance the immune response to a tumour in a mouse model, manifested by increased tumour infiltration by lymphocytes. The combination is highly advantageous, providing a new and enhanced treatment option for many cancer patients.

Of particular interest, an enhanced (e.g. a synergistic) response to therapy was seen in subjects with tumours which did not respond to therapy with a checkpoint inhibitor alone. The combination offers several advantages over therapies which use a combination of a checkpoint inhibitor with other cancer therapeutics such as chemotherapeutics, immunotherapeutics or oncolytic viruses. As noted above, CyPep-1 is well-tolerated in animal models, and thus has fewer side-effects than chemotherapeutics and immunotherapeutics, and is safer both for the patient and medical staff than an infectious oncolytic virus. The present invention thus provides a new and highly advantageous combination therapy for cancer. As described in WO 2011/092347, oligopeptidic compounds based on the sequence of CyPep-1 (SEQ ID NO: 1) may be prepared, including peptidomimetic compounds and peptide sequences comprising all or part of SEQ ID NO: 1, or sequence variants based on SEQ ID NO: 1. Further, the oligopeptidic compound may comprise one or more D-amino acids, and/or one more chemically modified amino acid residues.

Accordingly, in a first aspect the present invention provides an oligopeptidic compound comprising the amino acid sequence set forth in SEQ ID NO: 1, or an amino acid sequence having at least 85 % sequence identity thereto, for use in the treatment of a neoplastic condition, wherein said oligopeptidic compound has activity in inhibiting the growth and/or viability of neoplastic cells and said treatment comprises administering to a subject said oligopeptidic compound and an antibody which binds PD-1 or PD-L1 and blocks the interaction between PD-1 and PD-L1.

In another aspect the invention provides a kit for use in treating a neoplastic condition comprising an oligopeptidic compound comprising the amino acid sequence set forth in SEQ ID NO: 1, or an amino acid sequence having at least 85 % sequence identity thereto, and an antibody which binds PD-1 or PD-L1 and blocks the interaction between PD-1 and PD-L1.

In another aspect the invention provides a product comprising an oligopeptidic compound comprising the amino acid sequence set forth in SEQ ID NO: 1, or an amino acid sequence having at least 85 % sequence identity thereto, and an antibody which binds PD-1 or PD-L1 and blocks the interaction between PD-1 and PD-L1, for separate, simultaneous or sequential use in the treatment of a neoplastic condition in a subject.

As noted above, synergy has been observed between the oligopeptidic compound and the checkpoint inhibitor (i.e. in combination the two components act in synergy, or are synergistic). Thus, in certain embodiments, the oligopeptidic compound and checkpoint inhibitor (i.e. antibody which binds PD-1 or PD-L1 and blocks the interaction between PD-1 and PD-L1) are synergistically effective to treat the neoplastic condition. The oligopeptidic compound and checkpoint inhibitor may be administered to the subject in amounts such that a synergistic effect is obtained (in other words, the compound and inhibitor may be used in synergistic amounts). In particular, a synergistic effect may be seen such that the therapeutic effect of therapy with the combination of the oligopeptidic compound and the checkpoint inhibitor is greater than the cumulative effect of therapy with the same amount of the checkpoint inhibitor alone and therapy with the same amount of the oligopeptidic compound alone. The effect of therapy may be quantified based on e.g. change in tumour volume or any other quantifiable variable used in the art to measure the efficacy of a therapy for a neoplastic condition.

As detailed above, an oligopeptidic compound of SEQ ID NO: 1 is disclosed in WO 2011/092347. SEQ ID NO: 1 consists of a fragment of the tumour suppressor protein Conductin/Axin2 coupled to the C-terminus of the HIV-TAT cell-penetrating peptide. The aforementioned fragment of Conductin/Axin2 has the amino acid sequence set forth in SEQ ID NO: 2 (corresponding to amino acid numbers 13-27 of SEQ ID NO: 1) and the HIV-TAT cell-penetrating peptide has the amino acid sequence set forth in SEQ ID NO: 3 (corresponding to amino acid numbers 1-12 of SEQ ID NO: 1).

As used herein, the term "oligopeptidic compound" means a compound which is composed of amino acids or equivalent subunits, which are linked together by peptide or equivalent bonds. Thus, the term "oligopeptidic compound" includes peptides and peptidomimetics.

By "equivalent subunit" is meant a subunit which is structurally and functionally similar to an amino acid. The backbone moiety of the subunit may differ from a standard amino acid, e.g. it may incorporate one or more nitrogen atoms instead of one or more carbon atoms.

By "peptidomimetic" is meant a compound which is functionally equivalent or similar to a peptide and which can adopt a three-dimensional structure similar to its peptide counterparts, but which is not solely composed of amino acids linked by peptide bonds. A preferred class of peptidomimetics are peptoids, i.e. N-substituted glycines. Peptoids are closely related to their natural peptide counterparts, but they differ chemically in that their side chains are appended to nitrogen atoms along the molecule's backbone, rather than to the α-carbons as they are in amino acids.

Peptidomimetics typically have a longer half-life within a patient's body, so they are preferred in embodiments where a longer lasting effect is desired. This can help reduce the frequency at which the composition has to be re-administered. However, for bio-safety reasons a shorter half-life may be preferred in other embodiments; in those embodiments peptides are preferred.

Preferably, the oligopeptidic compound is an oligopeptide. The oligopeptidic compound may incorporate di-amino acids and/or β-amino acids. Most preferably, the oligopeptidic compound consists of α-amino acids.

An oligopeptide is a polymer formed from amino acids joined to one another by peptide bonds. As defined herein, an oligopeptide comprises at least three amino acids, though clearly an oligopeptidic compound for use according to the invention comprises more than three amino acids. An oligopeptidic compound or oligopeptide as defined herein has no particular maximum length, e.g. it may comprise up to 30, 40, 50 or 100 amino acids or more, but typically the prefix "oligo" is used to designate a relatively small number of subunits such as amino acids, i.e. less than 200, preferably less than 100, 90, 80, 70, 60 or 50 subunits. The oligopeptidic compound of the invention may thus comprise at least 23 and no more than 200 subunits. In embodiments it comprises at least 24, 25, 26 or 27 subunits. Alternatively defined it comprises no more than 50, 45, 40, 35, 30, 29, 28 or 27 subunits. The oligopeptidic compound may thus comprise a number of subunits in a range composed of any of the integers set out above for a minimum or maximum number of sub-units. Representative subunit ranges thus include 23-150, 23-100, 23-80, 23-50, 23-40, 23-30, 25-150, 25-100, 25-80, 25-50, 25-40, 25-30, 26-150, 26-100, 26-80, 26-50, 26-40, 26-30, 27-150, 27-100, 27-80, 27-50, 27-40, 27-30, 27-29 and 27-28.

An oligopeptidic compound as defined herein may be simply an oligopeptide, i.e. a polymer consisting of amino acids joined by peptide bonds. Alternatively, the oligopeptidic compound may comprise additional functional groups, conjugates, etc.

The oligopeptidic compound for use according to the present invention comprises the amino acid sequence set forth in SEQ ID NO: 1, or an amino acid sequence having at least 85 %, 90 % or 95 % sequence identity thereto. In a particular embodiment, the oligopeptidic compound comprises the amino acid sequence set forth in SEQ ID NO: 1. In another embodiment, the oligopeptidic compound consists of the amino acid sequence set forth in SEQ ID NO: 1, or an amino acid sequence having at least 85 %, 90 % or 95 % sequence identity thereto. In another embodiment, the oligopeptidic compound consists of the amino acid sequence set forth in SEQ ID NO: 1.

The level of sequence identity between two sequences (e.g. an oligopeptide sequence and the sequence set forth in SEQ ID NO: 1) may be determined by performing a sequence alignment. A sequence alignment may be performed using any suitable method, for instance a computer programme such as EMBOSS Needle or EMBOSS stretcher (both Rice, P. et al., Trends Genet. 16(6): 276-277, 2000) may be used for pairwise sequence alignments while Clustal Omega (Sievers, F. et al., Mol. Syst. Biol. 7:539, 2011) or MUSCLE (Edgar, R.C., Nucleic Acids Res. 32(5):1792-1797, 2004) may be used for multiple sequence alignments. Such computer programmes may be used with the standard input parameters, e.g. the standard Clustal Omega parameters: matrix Gonnet, gap opening penalty 6, gap extension penalty 1; or the standard EMBOSS Needle parameters: matrix BLOSUM62, gap opening penalty 10, gap extension penalty 0.5. Any other suitable parameters may alternatively be used.

The oligopeptidic compound for use according to the present invention may comprise only proteinogenic amino acids (i.e. the L-amino acids encoded by the standard genetic code). Alternatively the oligopeptidic compound for use according to the present invention may comprise one or more non-proteinogenic amino acids. For instance, the oligopeptidic compound for use according to the invention may comprise one or more D-amino acids (e.g. at least 1, 2, 3, 4, 5, 6, 7, or 8 D-amino acids), human-engineered amino acids or natural non-proteinogenic amino acids, e.g. amino acids formed through metabolic processes. Examples of non-proteinogenic amino acids which may be used include ornithine (a product of the urea cycle) and artificially-modified amino acids such as 9H-fluoren-9-ylmethoxycarbonyl (Fmoc)-, tert-Butyloxycarbonyl (Boc)-, and 2,2,5,7,8-pentamethylchromane-6-sulphonyl (Pmc)-protected amino acids, and amino acids having the carboxybenzyl (Z) group.

*In vitro* and/or *in vivo* stability of the oligopeptidic compounds of the invention may be improved or enhanced through the use of stabilising or protecting means known in the art, for example the addition of protecting or stabilising groups, incorporation of amino acid derivatives or analogues or chemical modification of amino acids. Such protecting or stabilising groups may for example be added at the N and/or C-terminus. An example of such a group is an acetyl group and other protecting groups or groups which might stabilise a peptide are known in the art.

A peptide consisting wholly of L-amino acids is known in the art as an L-peptide, while a peptide consisting wholly of D-amino acids is known in the art as a D-peptide. The term "inverso-peptide" is used to refer to a peptide with the same amino acid sequence as an L-peptide, but consisting wholly of D-amino acids (i.e. a D-peptide with the same sequence as a corresponding L-peptide). An inverso-peptide has a mirrored structure to its corresponding L-peptide (i.e. an L-peptide of the same amino acid sequence). Inverso-peptides can be advantageous for use in a clinical setting (relative to L-peptides) because they are not generally susceptible to degradation by serum proteases (due to their unnatural conformation inverso-peptides may not be recognised by protease enzymes). In a particular embodiment, the oligopeptidic compound for use according to the invention is an inverso compound, every amino acid of which is a D-amino acid. The oligopeptidic compound may in particular comprise or consist of a D-peptide consisting of the amino acid sequence set forth in SEQ ID NO: 1.

The oligopeptidic compound for use according to the present invention has activity in inhibiting the growth and/or viability of neoplastic cells. "Inhibiting the growth" of a cell means that any aspect of the growth of the cell, be that an increase in the size of the cell or in the amount and/or volume of its constituents, but more particularly an increase in the numbers of a cell, is reduced, more particularly measurably reduced. The term "growth" thus explicitly includes replication or reproduction of a cell. The rate of growth of a cell, e.g. in terms of the rate in increase of cell number, may be reduced. By way of representative example, growth (e.g. cell numbers, or rate of growth) may be reduced by at least 50, 60, 70, 80, 90 or 95 %. In certain cases, growth may be reduced by 100 %, i.e. growth may be completely inhibited and cease. Thus replication or reproduction of the cell may be reduced or inhibited. As described, the term "inhibit" includes any degree of reduction of growth.

Inhibition of cell growth may be identified by comparing the rate of growth of a control cell or cell population cultured under standard laboratory conditions and in the absence of an oligopeptidic compound of interest with the rate of growth of an identical or corresponding cell or cell population cultured in the presence of an oligopeptidic compound of interest but in otherwise identical conditions to the control cell or cell population. The rate of cellular replication or reproduction may in particular be assessed by determining cell numbers at a chosen time point. A reduction in cell number in the population cultured in the presence of the oligopeptidic compound relative to the number of cells in the control population indicates that the oligopeptidic compound has activity in inhibiting cell growth. Cell number (and thus growth or otherwise) may be determined by cell counting, e.g. using a haemocytometer.

"Inhibiting the viability" of a cell includes any effect which reduces the viability of a cell, or which renders it less likely to survive or non-viable. The viability of a cell may be viewed as the ability of a cell to survive under given conditions. Inhibition of viability of a cell in particular includes killing or destroying the cell, i.e. causing it to die. Cell death may be assessed by any standard laboratory technique. For instance, failure of a cell or cell population to grow, including to replicate, or to utilise or assimilate nutrients, may be considered indicative of cell death (i.e. lack of viability). Cell viability may also be assessed by monitoring morphological changes to the cell, or to tissue in which the cell is contained e.g. a tumour. Morphological changes may be analysed by microscopy, for example necrosis or cell lysis may be evident upon visual analysis of cells or tissue, indicating a lack of viability. Typically, a cell can be considered dead if cell membrane integrity is lost.

Inhibition of viability may for instance be identified by comparing the viability of a control cell or cell population incubated under standard laboratory conditions and in the absence of an oligopeptidic compound of interest with the viability of an identical or corresponding cell or cell population incubated in the presence of an oligopeptidic compound of interest but otherwise under identical conditions to the control cell or cell population. Cell viability is commonly assessed using a crystal violet assay, as known to the skilled person. In such an assay, a cellular monolayer adherent to a surface (e.g. a culture plate) is contacted (or not) with a compound of interest. Cell death leads to detachment of cells from the surface. Following contacting with the compound of interest, the monolayer is washed to remove detached cells and then stained with crystal violet, which binds proteins and DNA and thus stains cells. The level of staining can be used to determine viability, i.e. if a cell population contacted with a compound of interest is stained less than a control population, the compound of interest can be considered to inhibit the viability of cells. The level of crystal violet staining of a cell population may be determined visually (simply by eye) or quantitatively by dye extraction using methanol, followed by determination by spectroscopy of the optical density of the methanol-extracted dye at 570 nm.

Many other methods for determining the viability or growth of neoplastic cells are well known in the art, and many routine assays are available to determine if a cell is alive (viable) or dead. One option is to visually assess cells of interest for morphologies characteristic of cell death, e.g. necrotic or apoptotic bodies, membrane blebs, nuclear condensation and cleavage of DNA into regularly sized fragments, rupturing of cell membranes and leakage of cell contents into the extracellular environment. Other methods exploit the characteristic loss of cell membrane integrity in dead cells. Membrane-impermeable dyes (e.g. trypan blue and propidium iodide) are routinely used to assess membrane integrity. These dyes are excluded from intact cells and so no staining occurs in such cells. If cell membrane integrity is compromised, these dyes can access the cells and stain intracellular components. Alternatively, or in addition, dyes that only stain cells with intact membranes may be used to give an indication of the viability of a cell. The LIVE/DEAD cell viability assay available from Thermo Fisher Scientific is an assay that uses two dyes of different colours, one to stain dead cells, the other to stain live cells, thus enabling each to be identified. Examples of suitable live cell-specific dyes include calcein AM (green) and C12-resazurin (red); examples of suitable dead cell-specific dyes include ethidium homodimer-1 (red), propidium iodide (red) and SYTOX Green. Another approach to assessing membrane integrity is to detect the release of cellular components into the culture media, e.g. lactate dehydrogenase.

A still further option is to measure the metabolism of the cell. This can be done routinely in a number of ways, for instance the levels of ATP can be measured. Only living cells with intact membranes can synthesise ATP and because ATP is not stored in cells, levels of ATP drop rapidly upon cell death. Monitoring ATP levels therefore gives an indication of the status of the cell. A yet further option is to measure the reducing potential of the cell. Viable cells metabolising nutrients produce reducing agents (e.g. NADH and NADPH) and accordingly by applying a marker that gives different outputs whether in reduced or oxidised form (e.g. a fluorescent dye) to the cell, the reducing potential of the cell can be assessed. Cells that lack the ability to reduce the marker can be considered to be dead. The MTT and MTS assays are convenient examples of this type of assay.

As noted above, the oligopeptidic compound for use according to the present invention has activity in inhibiting the growth and/or viability of neoplastic cells. Thus the oligopeptidic compound may have activity in inhibiting the growth of neoplastic cells, it may have activity in inhibiting the viability of neoplastic cells or it may have activity in inhibiting the growth of neoplastic cells and in inhibiting the viability of neoplastic cells. Preferably the oligopeptidic compound has activity in inhibiting the viability of neoplastic cells, more preferably in inhibiting the growth and viability of neoplastic cells (as will be apparent, a compound which has activity in inhibiting the viability of neoplastic cells is likely to have activity in inhibiting their growth as well, though the reverse is not necessarily the case).

The term "neoplastic cell" as used herein refers to a cell which displays abnormal, excessive growth relative to a healthy cell. A neoplastic cell is derived from a neoplasm. A neoplasm is a tissue growth which grows in an abnormal and excessive manner, uncoordinated with that of surrounding healthy tissue. The term "neoplasm" encompasses cancer, and in particular a neoplastic cell may be a cancer cell. Thus the oligopeptidic compound for use according to the invention has activity in inhibiting the growth and/or viability of cancer cells. A neoplastic cell divides in an unchecked manner, and may be "immortal", that is to say telomerase-expressing and hence able to continue dividing ad *infinitum*, rather than dying or becoming senescent as does a healthy cell after reaching its Hayflick limit. The skilled person is able to determine whether a particular cell is neoplastic or healthy. Neoplastic cells often display distinguishing histological features enabling their identification, e.g. large and irregular nuclei and abnormalities within the cytoplasm. Determination of whether a cell is neoplastic may also be performed by genetic testing.

The oligopeptidic compound for use according to the invention has activity in inhibiting the growth and/or viability of both *in vivo* and *in vitro* neoplastic cells. Determination of this activity may conveniently be performed *in vitro* using a suitable cell line. Many laboratory cell lines are neoplastic, which due to their "immortality" are convenient for research uses. Any such neoplastic cell line may be used to determine the activity of a compound of interest, e.g. the cell lines A172 (human glioblastoma), GAMG (human glioblastoma), U87 (human glioblastoma), 4T1 (murine mammary carcinoma), HOS (human osteosarcoma) and MC38 (murine colon carcinoma). Many others are also known to the skilled person. Such cells may be obtained from any suitable source, e.g. a cell depository such as the ATCC (USA). The activity of a compound of interest is preferably determined using mammalian neoplastic cells. Human neoplastic cells may be used.

Neoplastic cells may also be obtained from a subject, e.g. a human cancer patient. Neoplastic cells may be surgically removed from a cancer patient and the activity of an oligopeptidic compound of interest tested thereupon. Thus the neoplastic cells may be from a neoplastic cell line, or derived from a clinical sample or veterinary sample. The neoplastic cells may be derived from a tumour, and may be benign or malignant. If the neoplastic cell is a cancer cell it may be from any cancer. Cancers are described in more detail below. In particular, the oligopeptidic compound for use according to the present invention has activity in inhibiting the growth and/or viability of human cancer cells.

In a particular embodiment, the oligopeptidic compound for use according to the present invention is selectively cytotoxic towards cancer cells. The term "cytotoxic" as used herein has essentially the same meaning as "inhibiting the viability of" as described above. In other words, the oligopeptidic compound selectively inhibits the viability of, or kills, cancer cells (or more preferably inhibits the viability of neoplastic cells generally).

A compound can be said to be selectively cytotoxic towards cancer cells if it has a greater cytotoxic effect against cancer cells than against non-cancerous cells, in particular if it has a greater cytotoxic effect against cancer cells than against healthy cells. Preferably the oligopeptidic compound for use according to the current invention has no or minimal effect on healthy, non-cancerous cells, but is cytotoxic towards cancer cells. In this way undesirable cytotoxic effects on non-cancerous cells may be avoided, thus reducing toxicity and undesirable side effects in patients to whom the oligopeptidic compound is administered.

Methods by which the effect of a compound of interest on cell growth and viability may be analysed are described above. Whether a compound of interest is selectively cytotoxic against cancer cells may be determined by the same method. However, rather than comparing the viability of a neoplastic cell population exposed to the compound of interest to that of a corresponding cell population not exposed to the compound of interest, the viability of a cancer cell population contacted with a compound of interest is compared to the viability of a population of healthy cells contacted with a compound of interest. If, following contacting with a compound of interest under identical conditions, the viability of the cancer cell population has been reduced more than the viability of the population of healthy cells, the compound of interest can be said to be selectively cytotoxic towards cancer cells.

The oligopeptidic compound for use according to the invention may also have activity in inhibiting the growth and/or viability of microbial cells, in particular bacterial cells. That is to say that the oligopeptidic compound may in particular have bacteriostatic or bacteriocidal activity. Antibacterial activity of the oligopeptidic compound may be determined by any standard method of antibiotic sensitivity testing. Such methods include e.g. disc diffusion (described in WO 00/55357) and are well known in the art.

Antibacterial activity of an oligopeptidic compound of interest may be determined by testing its activity against any bacterial species. Gram-positive and Gram-negative species are both suitable, including both pathogenic and non-pathogenic species. Exemplary species against which the antibacterial activity of a compound of interest may be determined include *Escherichia coli*, *Staphylococcus aureus* and *Enterococcus faecalis.* The oligopeptidic compound for use according to the invention may also have antimicrobial activity against other forms of microbe, e.g. archaea and fungi. Such activity may be tested analogously to the activities described above. Cancer patients are more susceptible to microbial infection than the population at large, due to such factors as weakness caused by the malignancy itself and damage to the immune system due to chemotherapy or other aggressive treatments. Antibacterial activity of the oligopeptidic compound is therefore highly advantageous as its administration offers protection against infection to cancer patients, as well as being therapeutically active against their cancer.

An oligopeptidic compound as described herein may be synthesised by the skilled person using standard biochemical techniques. If the oligopeptidic compound is an L-peptide comprising only proteinogenic amino acids, it may be synthesised by recombinant DNA technology. That is to say, a DNA sequence encoding the oligopeptidic compound may be cloned and introduced into an expression vector. A DNA sequence encoding an oligopeptidic compound for use according to the invention comprises or consists of a nucleotide sequence which encodes the amino acid sequence set forth in SEQ ID NO: 1, or an amino acid sequence having at least 85 %, 90 % or 95 % sequence identity thereto. Such a nucleotide sequence may be generated and synthesised by the skilled person without difficulty.

A DNA sequence encoding the oligopeptidic compound described herein may be generated by amplification from a template, e.g. by PCR, or by artificial gene synthesis, using standard methods known in the art. The DNA sequence encoding the oligopeptidic compound may then be introduced into an expression vector, using standard molecular cloning techniques such as restriction enzymes or Gibson assembly. Suitable expression vectors are known in the art. The expression vector may then be introduced into a cellular expression system using standard techniques. Suitable expression systems may include bacterial cells and/or eukaryotic cells such as yeast cells, insect cells or mammalian cells. Given that the oligopeptidic compound described herein may be toxic to bacterial cells (as discussed above), a eukaryotic cell may be a more appropriate cellular expression system for production of the oligopeptidic compound.

Instead of a cellular expression system, a cell-free, *in vitro* protein expression system may be used to synthesise an L-peptide compound for use according to the invention. In such a system a nucleotide sequence encoding the oligopeptidic compound is transcribed into mRNA, and the mRNA translated into a protein, *in vitro.* Cell-free expression system kits are widely commercially available, and can be purchased from e.g. Thermo Fisher Scientific (USA).

Oligopeptidic compounds for use according to the invention may alternatively be chemically synthesised in a non-biological system. Oligopeptidic compounds which comprise D-amino acids or other non-proteinogenic amino acids may in particular be chemically synthesised, since biological synthesis is generally not possible in this case. Liquid-phase protein synthesis or solid-phase protein synthesis may be used to generate polypeptides which may form or be comprised within the oligopeptidic compounds for use in the invention. Such methods are well-known to the skilled person, who can readily produce oligopeptidic compounds using appropriate methodology common in the art.

The present invention provides an oligopeptidic compound as described above for use in the treatment of a neoplastic condition in a subject. A "neoplastic condition" as defined herein is a medical condition characterised by the development of one or more neoplasms. Thus non-malignant (i.e. benign), pre-malignant and malignant neoplasms (i.e. cancer) are encompassed by the term "neoplastic condition". The subject to which the oligopeptidic compound and checkpoint inhibitor are administered, according to the present invention, is a subject suffering from a neoplastic condition.

According to the present invention the oligopeptidic compound defined above is used in combination with a checkpoint inhibitor to treat a neoplastic condition, wherein the checkpoint inhibitor is an antibody which binds PD-1 or PD-L1 and blocks the interaction between PD-1 and PD-L1.

Immune checkpoints are regulators of the immune system which function to promote antigen-specific activation of immune cells and to enable self-tolerance, thus supporting immune activity against antigenic targets and preventing auto-immune disease and aberrant immune system activity against host tissues. Immune checkpoints may be stimulatory or inhibitory. Stimulatory immune checkpoints act to enhance immune cell activity against antigenic targets, by stimulating proliferation and effector responses when bound by their cognate ligand or agonist. Examples of stimulatory immune checkpoints include CD28, which acts as a co-stimulator for T-cell activity and initiates proliferation of T-cells upon binding to its ligands, CD80 and CD86.

Inhibitory immune checkpoints down-regulate or inhibit immune cell function upon binding by their cognate ligand or agonist, promoting self-tolerance and preventing autoimmune activity or excessive and aberrant immune responses with the potential to cause damage to the host, such as cytokine storms. However, as discussed above, activation of inhibitory immune checkpoints can prevent the immune system from targeting cancer cells. As detailed above, examples of such inhibitory immune checkpoints include PD-1 and CTLA-4. A checkpoint inhibitor as defined herein (and generally in the art) is an agent which inhibits the activity of an inhibitory immune checkpoint. With the exception of the paragraph above where its meaning is explicitly defined, throughout the present disclosure the term "immune checkpoint" means an inhibitory immune checkpoint.

As defined herein a checkpoint inhibitor refers to any agent which binds an immune checkpoint or immune checkpoint ligand and acts directly to prevent activation of the immune checkpoint. Thus a checkpoint inhibitor may be an antagonist of an immune checkpoint. All currently-available checkpoint inhibitors act by blockading their target immune checkpoint, i.e. binding to it or its ligand and thus preventing the interaction between checkpoint and ligand (a mechanism known as immune checkpoint blockade). The checkpoint inhibitor for use in the invention in combination with the oligopeptidic compound is an antibody which binds PD-1 or PD-L1 and blocks the interaction between PD-1 and PD-L1. Ideally a checkpoint inhibitor should cause cancer cells to be exposed to the immune system without causing that same system to attack healthy tissue.

An antibody, as referred to herein, may be a natural or synthetic antibody, or a fragment thereof. The term "antibody" is used broadly herein to include any type of antibody. This includes not only native antibody molecules but also modified, synthetic or recombinant antibodies, as well as fragments thereof. An antibody may thus be any molecule or entity or construct having antibody-based binding region(s), that is a binding domain(s) which is/are derived from an antibody. Accordingly, an antibody may alternatively be defined as a binding molecule comprising an antigen-binding domain obtained or derived from an antibody. The antibody may be of, or may be derived from/based on, an antibody of any convenient or desired species, class or sub-type. As noted above, the antibody may be natural, derivatised or synthetic. It may be monoclonal or polyclonal. Thus the antibody may bind to a single epitope or it may be a mixture of antibodies (or antibody molecules) binding to different epitopes.

Accordingly, the checkpoint inhibitor for use according to the present invention is a binding molecule comprising an antigen-binding domain from an antibody specific for (or directed against) PD-1 or PD-L1, and which blocks the interaction between PD-1 and PD-L1. Examples of such "antibodies" (i.e. antibody-based binding molecules) include monoclonal and polyclonal antibodies, antibody fragments including Fab, Fab', F(ab')₂ and Fv fragments and any fragment lacking an Fc region, chimeric (e.g. humanised or CDR-grafted) antibodies, single chain antibodies (e.g. scFv antibodies), antibodies identified or obtained from phage display, etc. In a particular embodiment the checkpoint inhibitor is a monoclonal antibody.

The checkpoint inhibitor used according to the present invention inhibits the activity of PD-1, by blocking the interaction between PD-1 and PD-L1 (the interaction between PD-1 and PD-L2 may also be blocked), thus preventing PD-1 activation (as described above, PD-1 activation inhibits T-cell effector functionality). A checkpoint inhibitor which blocks the interaction between PD-1 and PD-L1 binds to one of these proteins and prevents interaction between the two proteins from taking place. Thus a checkpoint inhibitor which blocks the interaction between PD-1 and PD-L1 may bind to PD-1 or may bind to PD-L1. In particular, such a checkpoint inhibitor may bind to the PD-L1 binding site of PD-1, or the PD-1 binding site of PD-L1. It may be advantageous to use a checkpoint inhibitor which binds PD-1 to block the interaction between PD-1 and its ligands, in order to block interactions between PD-1 and both PD-L1 and PD-L2.

In particular embodiments of the invention, the checkpoint inhibitor which blocks the interaction between PD-1 and PD-L1 (and optionally PD-L2) is an antibody (preferably a monoclonal antibody, or a derivative or fragment thereof) which binds PD-1. In other embodiments, the checkpoint inhibitor which blocks the interaction between PD-1 and PD-L1 is an antibody (preferably a monoclonal antibody, or a derivative or fragment thereof) which binds PD-L1. A number of such antibodies are known in the art, for instance Nivolumab (Bristol-Myers Squibb), a human monoclonal anti-PD1 IgG4 antibody; Pembrolizumab, a humanized IgG4 anti-PD-1 antibody (Merck); Atezolizumab, a fully humanised anti-PD-L1 antibody (Genentech); and Durvalumab, a human anti-PD-L1 antibody (Medlmmune/AstraZeneca), have all received regulatory approval and may be used according to the present invention. Many other such antibodies are currently in development/trials, such as Tislelizumab, a humanised anti-PD-1 antibody (BeiGene); and Avelumab, a fully human anti-PD-L1 antibody (Pfizer/Merck), and may also be used according to the present invention.

As detailed above, PD-1 and CTLA-4 are expressed on T-cells. PD-1 and CTLA-4 inhibition is designed to promote T-cell activity, and so when targeting PD-1 or CTLA-4 with an antibody, it may be preferable that binding of the antibody to its target does not initiate antibody-dependent cellular cytotoxicity (ADCC), which could cause the death of the target T-cell. ADCC is primarily mediated by natural killer (NK) cells, which express Fc receptors (such as CD16) which recognise and bind the Fc (i.e. constant) domains of antibodies bound to target antigens. Binding of an Fc receptor of an NK cell to the Fc domain of an antigen-bound antibody leads to activation of the NK cell, which releases cytotoxic agents which kill the cell to which the antibody is bound.

Antibodies able to bind target cells without inducing ADCC may be of a particular IgG sub-class which is not associated with ADCC activity, or may be rationally designed by introducing point mutations to inhibit Fc receptor binding. Such rational design is straightforward for the skilled person. For instance, mutation of position 228 in the human IgG4 constant region may prevent Fc receptor binding of the antibody. Thus Nivolumab and Pembrolizumab (both of which are human IgG4 antibodies, as mentioned above) both contain an S228P mutation in their constant regions which prevents Fc receptor binding, meaning neither antibody mediates ADCC. Any antibody against PD-1 for use as a checkpoint inhibitor according to the present invention may comprise the same or an equivalent mutation. By equivalent mutation is meant a mutation at a different residue (or a corresponding residue in the constant region of a different antibody isotype) which has the same effect, i.e. inhibition of Fc receptor binding.

However, in other contexts it may be preferred that the checkpoint inhibitor is able to mediate ADCC. The anti-CTLA-4 antibody Ipilimumab has been shown to mediate ADCC against Treg cells, mediated by non-classical CD16-expressing monocytes, thus providing a second mechanism of preventing immune effector cell down-regulation (Romano et al., PNAS 112(19) 6140-6145, 2015).

Though less prominent, other immune checkpoints in addition to PD-1 are also known and may additionally be targeted by a checkpoint inhibitor. For instance LAG-3 (also known as CD223) is an immune checkpoint expressed by T-cells which binds MHC Class II proteins (with higher affinity than does CD4). Binding of LAG-3 to MHC Class II down-regulates cellular proliferation and effector functionality. LAG-3 is also believed to play a role in activating the immunosuppressive role of Treg cells. An agent which inhibits LAG-3 activation may be used as a checkpoint inhibitor in accordance with the current invention. Such an agent may block the interaction between LAG-3 and MHC Class II. In particular, such an agent may be an antibody which binds LAG-3, a number of which are in development, such as BMS-986016 (Bristol-Myers Squibb).

In a further alternative approach an inhibitor of killer cell immunoglobulin-like receptor (KIR) may be used as an additional checkpoint inhibitor. KIR is a receptor on NK cells that down-regulates NK cell cytotoxic activity. HLA class I allele-specific KIR receptors are expressed in cytolytic (CD56dimCD16+) NK cells, while CD56brightCD16- NK subset lacks these KIRs. Along these lines, inhibitory KIRs seem to be selectively expressed in the peritumoral NK cell infiltrate and thus seem to be a checkpoint pathway coopted by tumours, similar to PD-L1. As such, inhibition of specific KIRs should cause sustained *in vivo* activation of NK cells. In particular, antibodies against KIR may be used as a checkpoint inhibitor in accordance with the present invention. For example Lirilumab (Bristol-Myers Squibb) is a fully human monoclonal antibody to KIR which may be used as a checkpoint inhibitor according to the invention.

Other immune checkpoints which may be additionally targeted by checkpoint inhibitors according to the present invention (to prevent their activation, for instance by blocking their interaction with their cognate ligands) include B7-H3 (also known as CD276), BTLA (also known as CD272), VISTA and TIM-3 (also known as HAVCR2). Where appropriate, the ligands of these checkpoints may also be targeted by checkpoint inhibitors in order to block interaction of the ligand with its immune checkpoint receptor. For instance, the ligands of TIM-3 may be targeted by checkpoint receptors. The ligands of TIM-3 include galectin-9 and phosphatidylserine (PS), which is a phospholipid present in the inner envelope of the plasma membrane of healthy cells. PS is translocated to the outer envelope of the membrane during apoptosis, where it binds to TIM-3 on T-cells, suppressing the excess immune activation that would otherwise occur during processing and clearance of decaying cell matter. Externalisation of PS indirectly stimulates macrophages, resulting in suppression of dendritic cell antigen presentation. Like PD-L1, externalised PS is aberrantly expressed by some tumour cells and tumour-derived microvesicles. Thus, PS is believed to be exploited by tumours to prevent adaptive anti-tumour immunity (Birge et al., Cell Death & Differentiation 23, 962-978, 2016). PS may thus be targeted by checkpoint inhibitors to block its interaction with TIM-3, for instance using an anti-PS antibody. An example of such an antibody is Bavituximab (Oncologie Inc.), which is currently in development.

Thus as set out above, the checkpoint inhibitor used according to the invention is an antibody which binds PD-1 or PD-L1 and blocks the interaction between PD-1 and PD-L1. In particular embodiments, more than one checkpoint inhibitor may be used in combination with the oligopeptidic compound. For instance, two or more different checkpoint inhibitors, which each inhibit the activation of different immune checkpoints, may be used. For instance the checkpoint inhibitor which blocks PD-1 activation may be used in combination with a checkpoint inhibitor which blocks CTLA-4 activation. Use of multiple checkpoint inhibitors in combination has previously been shown to yield improvement in treatment outcomes in some cancers relative to the use of any single checkpoint inhibitor (Wolchok et al., N Engl J Med 369: 122-133, 2013).

According to the present invention, the neoplastic condition may be treated by separate, simultaneous or sequential administration of the oligopeptidic compound and checkpoint inhibitor to the subject. By "separate" administration, as used herein, is meant that the oligopeptidic compound and the checkpoint inhibitor are administered to the subject at the same time, or at least substantially the same time, but by different administrative routes. "Simultaneous" administration, as used herein, means that the oligopeptidic compound and the checkpoint inhibitor are administered to the subject at the same time, or at least substantially the same time, by the same administrative route. By "sequential" administration, as used herein, is meant that the oligopeptidic compound and the checkpoint inhibitor are administered to the subject at different times. In particular, administration of the first therapeutic agent is completed before administration of the second therapeutic agent commences. When administered to a subject sequentially, the first and second therapeutic agent may be administered by the same administrative route or by different administrative routes.

Administration of the oligopeptidic compound and/or the checkpoint inhibitor may be performed repeatedly (i.e. two or more times) during the course of treatment of a subject. For instance, the subject may receive a number of cycles of treatment, in which both the oligopeptidic compound and the checkpoint inhibitor are administered. Alternatively, the subject may receive a single dose of one of the therapeutic agents and repeated doses of the other.

If multiple checkpoint inhibitors are administered to the subject in combination with the oligopeptidic compound, the two or more checkpoint inhibitors may be administered separately, simultaneously or sequentially to one another.

The oligopeptidic compound and the checkpoint inhibitor may be administered by any suitable route. Such a route may be determined by the skilled physician and may be dependent on the condition to be treated. Possible routes of administration include oral, rectal, nasal, topical, vaginal and parenteral administration. Oral administration as used herein includes buccal and sublingual administration. Topical administration as used herein includes transdermal administration. Parenteral administration as defined herein includes subcutaneous, intramuscular, intravenous, intraperitoneal and intradermal administration. The oligopeptidic compound in particular may be administered to the subject for systemic delivery, for example via an oral or parenteral route of administration, or be administered locally to the site of the neoplastic condition to be treated, e.g. locally to a tumour. Possible routes of local administration include topical administration, delivery by direct administration e.g. by injection or infusion to the site of the neoplasm (e.g. tumour), and inhalation, depending of course on the site of the cancer (tumour). In a particular embodiment the oligopeptidic compound is administered to the subject by intratumoural administration.

As noted above, the oligopeptidic compound may be administered to the subject via the same route or a different route to that by which the checkpoint inhibitor is administered, and if two or more checkpoint inhibitors are administered to the subject, the two or more checkpoint inhibitors may be administered to the subject via the same or different routes. In a particular embodiment, the checkpoint inhibitor is administered parenterally to the subject. For instance, the checkpoint inhibitor may be administered to the subject intravenously.

The oligopeptidic compound and checkpoint inhibitor are preferably administered to the subject within a pharmaceutical composition. The pharmaceutical composition may take any appropriate form known in the art, for example a liquid form such as a solution, suspension, syrup or emulsion, or a solid form such as a tablet, capsule, coated tablet, powder, pellet or granule. The pharmaceutical composition may take the form of a cream, ointment or salve, or an inhalant, lyophilisate or spray, or any other style of composition commonly used in the art. It may be provided e.g. as a gastric fluid-resistant preparation and/or in sustained-action form. It may be a form suitable for oral, parenteral, topical, rectal, genital, subcutaneous, transurethral, transdermal, intranasal, intraperitoneal, intramuscular and/or intravenous administration and/or for administration by inhalation. The oligopeptidic compound and checkpoint inhibitor may be administered within a single pharmaceutical composition, or each may be administered within a separate pharmaceutical composition.

The pharmaceutical composition preferably also contains one or more pharmaceutically-acceptable diluents, carriers or excipients. Suitable pharmaceutically-acceptable diluents, carriers and excipients are well known in the art. For instance, suitable excipients include lactose, maize starch or derivatives thereof, stearic acid or salts thereof, vegetable oils, waxes, fats and polyols. Suitable carriers or diluents include carboxymethylcellulose (CMC), methylcellulose, hydroxypropylmethylcellulose (HPMC), dextrose, trehalose, liposomes, polyvinyl alcohol, pharmaceutical grade starch, mannitol, lactose, magnesium stearate, sodium saccharin, talcum, cellulose, glucose, sucrose (and other sugars), magnesium carbonate, gelatine, oil, alcohol, detergents and emulsifiers such as polysorbates. Stabilising agents, wetting agents, sweeteners etc. may also be used.

Liquid pharmaceutical compositions, whether they be solutions, suspensions or other like form, may include one or more of the following: sterile diluents such as water, saline solution (preferably physiological, i.e. isotonic), Ringer's solution, fixed oils such as synthetic mono- or diglycerides which may serve as a solvent or suspending medium, polyethylene glycols, glycerine, propylene glycol or other solvents; antibacterial agents such as benzyl alcohol or methyl paraben; antioxidants such as ascorbic acid or sodium bisulfite; chelating agents such as EDTA; buffers such as acetates, citrates or phosphates and agents for the adjustment of tonicity such as sodium chloride or dextrose. A parenteral preparation can be enclosed in ampoules, disposable syringes or multiple dose vials made of glass or plastic. An injectable pharmaceutical composition is preferably sterile.

The oligopeptidic compound and the checkpoint inhibitor (or pharmaceutical compositions comprising them) may be administered to the subject in a manner appropriate to the neoplastic condition to be treated. The quantity and frequency of administration will be determined by such factors as the condition of the patient, and the type and severity of the patient's disease, although appropriate dosages may be determined by clinical trials. Conveniently the oligopeptidic compound and/or the checkpoint inhibitor may be provided to a subject in a daily, weekly or monthly dose, or a dose in an intermediate frequency, e.g. a dose may be provided every 2, 3, 4, 5 or 6 days, every 2, 3, 4, 5 or 6 weeks, every 2, 3, 4, 5 or 6 months, annually or biannually. As noted above, the same dosage regime or different dosage regimes may be used for administration to the subject of the oligopeptidic compound and the checkpoint inhibitor.

Doses may be administered in amounts dependent on the size of the subject. The amount of oligopeptidic compound and checkpoint inhibitor administered according to the combination therapy of the invention is therapeutically effective. The oligopeptidic compound may be administered in doses of from 10 µg/kg to 100 mg/kg body mass, e.g. 10 µg/kg to 50 mg/kg body mass, 10 µg/kg to 10 mg/kg body mass, 10 µg/kg to 5 mg/kg body mass, 10 µg/kg to 2.5 mg/kg body mass, 100 µg/kg to 5 mg/kg body mass, 100 µg/kg to 2.5 mg/kg body mass, 500 µg/kg to 5 mg/kg body mass, or 1 mg/kg to 5 mg/kg body mass. In a particular embodiment, the oligopeptidic compound is administered in a dose of about 2 mg/kg body mass, e.g. 1 mg/kg to 2.5 mg/kg body mass, 1.5 mg/kg to 2.5 mg/kg body mass or 1.8 mg/kg to 2.2 mg/kg body mass. The skilled clinician will be able to calculate an appropriate dose for a patient based on all relevant factors, e.g. age, height, weight, the condition to be treated and its severity.

The checkpoint inhibitor may be administered at the same dose as the oligopeptidic compound, or may be administered at a higher dose or, in particular, a lower dose to the oligopeptidic compound. For instance, the checkpoint inhibitor may be administered at a dose of from 100 µg/kg to 100 mg/kg body mass, e.g. 500 µg/kg to 50 mg/kg body mass or 1 mg/kg to 10 mg/kg body mass. Exemplary doses include 1 mg/kg body mass, 2 mg/kg body mass, 3 mg/kg body mass, 4 mg/kg body mass, 5 mg/kg body mass, 6 mg/kg body mass, 7 mg/kg body mass, 8 mg/kg body mass, 9 mg/kg body mass and 10 mg/kg body mass. The checkpoint inhibitor may be administered at a fixed dose, e.g. from 100 mg to 1.5 g. Exemplary doses of checkpoint inhibitor include 100 mg, 200 mg, 240 mg, 250 mg, 300 mg, 400 mg, 480 mg, 500 mg, 600 mg, 700 mg, 800 mg, 900 mg, 1000 mg, 1100 mg, 1200 mg, 1300 mg, 1400 mg and 1500 mg.

Suitable dosage regimes for many checkpoint inhibitors are known. E.g. nivolumab, when used alone, is administered following a dosage regime of 240 mg IV every 2 weeks or 480 mg IV every 4 weeks; ipilimumab, when used alone in melanoma therapy is administered following a dosage regime of 3 mg/kg IV every 3 weeks. These and many other such checkpoint inhibitor dosage regimes are known to the skilled person and may be found within the licensing approvals issued by regulatory bodies such as the FDA and EMA.

As noted above, the subject to which the oligopeptidic compound and checkpoint inhibitor are administered is a subject suffering from a neoplastic condition. The subject is an animal, preferably a mammal. The subject may be a rodent, such as a mouse, rat, rabbit or guinea pig. The subject may be a pet animal, such as a cat or dog, or a farm animal, such as a horse, cow, sheep, pig or goat. The subject may be a wild animal, e.g. an animal in a zoo or game park. In a particular embodiment the subject is a primate, such as a monkey or an ape. Most preferably the subject is a human. Thus the therapy disclosed herein may be for veterinary or clinical purposes, but is preferably for clinical purposes, i.e. for the treatment of a human subject with a neoplastic condition (e.g. a cancer patient).

The term "treatment" as used herein refers broadly to any effect or step (or intervention) beneficial in the management of a clinical condition. Treatment may include reducing, alleviating, ameliorating, slowing the development of, or eliminating the condition or one or more symptoms thereof, which is being treated, relative to the condition or symptom prior to the treatment, or in any way improving the clinical status of the subject. A treatment may include any clinical step or intervention which contributes to, or is a part of, a treatment programme or regimen. Thus "treatment" as used herein encompasses curative treatment (or treatment intended to be curative), and treatment which is merely life-extending or palliative (i.e. designed merely to limit, relieve or improve the symptoms of a condition).

The oligopeptidic compound and checkpoint inhibitor according to the current invention are for use in the treatment of a neoplastic condition in a subject. As detailed above, a neoplastic condition may be benign or malignant. In a particular embodiment however, the neoplastic condition is a malignant condition, i.e. the oligopeptidic compound and checkpoint inhibitor are for treating cancer.

The cancer to be treated may be any cancer, and may be a primary tumour or a metastasis (i.e. a secondary cancer). Exemplary cancers which may be treated using the combination therapy disclosed herein include cervical cancer, anal cancer, vaginal cancer, vulvar cancer, penile cancer, melanoma, lung cancer, head and neck cancers, bladder cancer, kidney cancer, Hodgkin's lymphoma, squamous cell carcinomas and Merkel cell carcinoma. Such cancers may be diagnosed using standard techniques by the skilled person.

Rather than being defined based on its primary location or initiating tissue type, the cancer to be treated may alternatively be defined based on its genetic identity. In particular, the cancer to be treated using the combination therapy disclosed herein may be microsatellite instability-high and/or mismatch repair-deficient.

Microsatellites (also known as "short tandem repeats") are DNA sequences scattered throughout the genome (including both coding and non-coding regions) consisting of a repeating unit sequence. An individual microsatellite generally comprises between 10 and 60 copies of the repeating unit, which range from 1 to 6 base pairs in length. Due to the repeating nature of microsatellites, DNA polymerases are much more prone to making mistakes in these regions than in other regions of the genome. In cells with a functional mismatch repair (MMR) system, the MMR machinery "proofreads" newly-synthesised DNA strands, correcting errors made by the polymerase. Cancer cells which have a defect in the MMR machinery are unable to correct these errors, and thus have a 100 to 1000-fold increase in point mutations within their microsatellites. This increase in mutation rate in microsatellites is known as microsatellite instability (MSI) (Dudley et al., Clin Cancer Res 22(4): 813-820, 2016). A "microsatellite instability-high" cancer is a cancer which demonstrates MSI. A "mismatch repair-deficient" cancer is a cancer lacking a functional MMR machinery.

MSI can be inherited or can spontaneously develop. Lynch syndrome is an autosomal dominant condition in which an individual has one or more germline mutations in genes encoding the MMR machinery, resulting in MMR deficiency. Lynch syndrome sufferers have a high risk of developing cancer. The oligopeptidic compound and checkpoint inhibitor for use according to the present invention may be used for the treatment of cancer in a patient suffering from Lynch syndrome. Non-inherited MSI is generally caused by epigenetic silencing of expression of one or more genes involved in MMR, or occasionally by loss-of-function mutations within these genes.

MSI may be identified in a cancer by genetic testing. Details of tests for MSI are set forth in Dudley *et al.* (supra). As detailed therein, a panel of five specific microsatellites are amplified in both tumour tissue and healthy tissue. A shift in size of at least two of the five microsatellites is considered diagnostic for MSI. As also detailed in Dudley *et al.,* MMR deficiency can be diagnosed by checking tumour samples for loss of expression of MMR machinery proteins by immunohistochemistry. Current guidelines recommend that tumours be screened for MSI by concurrent DNA-based MSI analysis, immunohistochemistry for MMR proteins, and screening for mutation of the *BRAF* gene, which encodes the serine/threonine kinase B-Raf. Mutation of *BRAF* is associated with some cases of Lynch syndrome/MSI.

MSI is associated with increased mutational load in cancers (due to the deficiency in the MMR machinery), resulting in increased production of neoantigens in cancer cells and thus an increased immune response to the cancer. MSI-high cancer cells are associated with increased PD-L1 expression relative to other cancers, due to their need to down-regulate immune expression to avoid T-cell-mediated destruction, and thus are considered particularly strong targets for checkpoint inhibitor therapy. In 2017 pembrolizumab (discussed above) was licensed by the FDA for treatment of MSI-high and/or MMR-deficient tumours. This was the first occasion on which a cancer drug was approved for use in the treatment of cancers based on a particular biomarker alone rather than the location in the body in which the tumour originated.

MSI is most associated with colorectal cancer, though is also associated with gastric cancer, endometrium cancer, ovarian cancer, hepatobiliary tract cancer, urinary tract cancer, brain cancer and skin cancers. The combination therapy disclosed herein may be used to treat any such MSI-high cancer.

In another embodiment, the combination therapy disclosed herein is used in treatment for a cancer associated with human papillomavirus (HPV). HPV is a DNA virus of the *papillomavirus* family. HPV is a sexually-transmitted infection which only affects humans. Some strains of HPV are oncogenic. HPV-mediated carcinogenesis occurs through the viral oncogenes E6 and E7 which, respectively, promote the degradation of the tumour suppressor protein p53 and bind and inhibit the tumour suppressor protein pRb (Narisawa-Saito & Kiyono, Cancer Science 98(10): 1505-1511, 2007). HPV is particularly associated with cervical cancer, anal cancer, penis cancer, vulva cancer, vaginal cancer and head and neck cancers including larynx cancer and oropharynx cancer.

The combination therapy disclosed herein thus may be used to treat cancer in a subject who is HPV-positive, in particular a cancer mentioned above as being particularly associated with HPV. Methods by which HPV may be detected in an individual are reviewed in Abreu et al., 2012 (Virology Journal 9: 262). Such methods generally rely on identification of HPV-associated DNA sequences, including by hybridisation (Southern blot) and amplification assays including qPCR and microarray-based assays. A number of kits for HPV detection are commercially available, including e.g. PapilloCheck^{®} (Greiner Bio-One, Austria).

The invention as described above may be seen as a method of treating a neoplastic condition in a subject, comprising administering an oligopeptidic compound and a checkpoint inhibitor to a subject in need thereof. Such a subject may be identified by a physician, and as described above is a subject suffering from a neoplastic condition. The oligopeptidic compound, checkpoint inhibitor, subject, treatment and neoplastic condition may each be as described above.

Similarly, the invention provided may be seen as the use of an oligopeptidic compound in the manufacture of a medicament for treating a neoplastic condition, wherein the treatment of said neoplastic condition comprises administering said medicament and a checkpoint inhibitor to a subject. Again, the oligopeptidic compound, checkpoint inhibitor, subject, treatment and neoplastic condition may each be as described above.

In another aspect, the invention provides a kit for use in treating a neoplastic condition comprising an oligopeptidic compound as defined above and a checkpoint inhibitor, wherein the checkpoint inhibitor is an antibody which binds PD-1 or PD-L1 and blocks the interaction between PD-1 and PD-L1, as set out above. The kit may comprise a first container comprising the oligopeptidic compound and a second container comprising the checkpoint inhibitor. Alternatively, the kit may comprise a single container comprising both the oligopeptidic compound and the checkpoint inhibitor. The oligopeptidic compound and checkpoint inhibitor may be provided in the kit in any suitable form. For instance, the oligopeptidic compound and/or checkpoint inhibitor may be provided in the form of a pharmaceutical composition, as described above. Neoplastic conditions and subjects are described above.

In another aspect, the invention provides a product comprising an oligopeptidic compound as defined above and a checkpoint inhibitor for separate, simultaneous or sequential use in the treatment of a neoplastic condition in a subject, wherein the checkpoint inhibitor is an antibody which binds PD-1 or PD-L1 and blocks the interaction between PD-1 and PD-L1, as set out above. The neoplastic condition, treatment and subject may be as defined above.

The present invention may be more fully understood from the non-limiting Examples below and in reference to the drawings, in which:

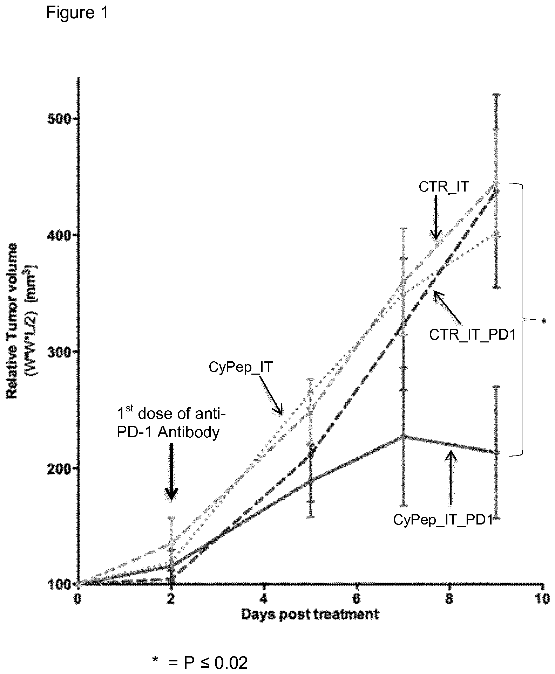

Figure 1 shows the effect on tumour volume in a mouse colon cancer model of treatment with a combination of CyPep-1 with an anti-PD-1 antibody relative to treatment with CyPep-1 alone or the antibody alone. Day 0 corresponds to the day on which the mice received the second and final CyPep-1 dose (or corresponding control), i.e. "post treatment" on the x-axis means post treatment with CyPep-1. The day on which the first dose of anti-PD-1 antibody (or equivalent control) was administered to the mice is indicated on the figure. Error bars indicate standard error of the mean (SEM).

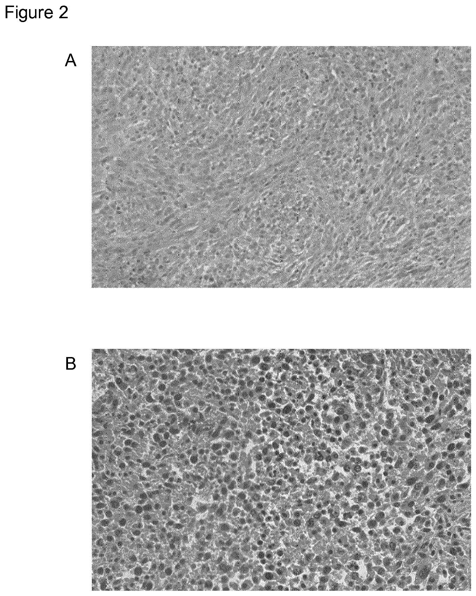

Figure 2 shows microscope images of tumours removed post-mortem from mice treated with anti-PD-1 antibody alone (A) and anti-PD-1 antibody in combination with CyPep-1 (B). As can be seen, the tumour from the mouse treated with the combination of anti-PD-1 antibody and CyPep-1 contains a much greater number of TILs (i.e. the cells with the larger, dark-stained nuclei) than does the tumour taken from the mouse treated with anti-PD-1 antibody alone.

### Examples

### Materials

The CyPep-1 peptide was synthesised by Bachem AG (Switzerland). CyPep-1 is an all D-amino acid peptide consisting of the amino acid sequence set forth in SEQ ID NO: 1. Antimouse PD-1 antibody was obtained from Bio X Cell (USA). The monoclonal antibody used was Clone RMP1-14, a rat antibody of isotype IgG2a, which is known to block binding of PD-L1 and PD-L2 to PD-1.

### Methods

Female C57/BL6N mice were shaved at the intended site of tumour inoculation. 4 days later the mice were implanted with 5 × 10⁵ MC38 colon carcinoma cells in a mixture of 50 % PBS and 50 % matrigel in a total injection volume of 100 µl.

Tumour sizes were measured by caliper. When the median tumour volume reached 100 mm³, 40 mice were randomised to four groups: 1) Ctr_IT; 2) Ctr IT_PD1; 3) CyPep_IT; and 4) CyPep_IT_PD1.

The Ctr_IT group were administered 0.05 ml/kg intratumoural PBS on days 1 and 2.

The Ctr_IT_PD1 group were administered 0.05 ml/kg/day intratumoural PBS on days 1 and 2, and 5 mg/kg intraperitoneal anti-PD-1 antibody on days 4, 8, 11 and 15. The anti-PD-1 antibody was administered in PBS at a concentration of 1 mg/ml.

The CyPep_IT group were administered 2 mg/kg CyPep-1 on days 1 and 2. Administration was intratumoural in PBS at a concentration of 40 mg/ml.

The CyPep_IT_PD1 group were administered 2 mg/kg intratumoural CyPep-1 in PBS at a concentration of 40 mg/ml on days 1 and 2; and 5 mg/kg intraperitoneal anti-PD-1 antibody on days 4, 8, 11 and 15, in PBS at a concentration of 1 mg/ml.

All groups were standardised prior to checkpoint inhibitor administration by excluding animals with tumours +/- >2X average at day three. Mice were sacrificed once a tumour volume of 1500 mm³ was reached, upon occurrence of tumour ulceration or 6 weeks after tumour injection. Tumours were removed from mice post-mortem. Average tumour volumes of the groups were analysed by unpaired two-tailed *t*-test, and the tumours were also histologically analysed.

### Results

As shown in Figure 1, no statistically-significant difference in tumour growth was seen in mice treated with CyPep-1 alone or the anti-PD-1 antibody alone compared to the control group which received only PBS. However, the mice which received both CyPep-1 and anti-PD-1 antibody showed significantly reduced tumour growth relative to the control (P=0.02). The mice which received both CyPep-1 and anti-PD-1 antibody demonstrated an average 52 % reduction in tumour volume relative to the control mice.

Histology results are shown in Figure 2. Tumours were analysed by microscopy, and it was found that tumours of mice treated with the combination of CyPep-1 and anti-PD-1 antibody (Fig. 2B) contained significantly higher numbers of tumour-infiltrating lymphocytes (TILs) than did the tumours of mice treated with anti-PD-1 antibody alone (Fig. 2A). TIL numbers are an indicator of immune activity against a tumour.

## Claims

1. An oligopeptidic compound comprising the amino acid sequence set forth in SEQ ID NO: 1, or an amino acid sequence having at least 85 % sequence identity thereto, for use in the treatment of a neoplastic condition,
wherein said oligopeptidic compound has activity in inhibiting the growth and/or viability of neoplastic cells,
and said treatment comprises administering to a subject said oligopeptidic compound in combination with an antibody which binds PD-1 or PD-L1 and blocks the interaction between PD-1 and PD-L1.

2. The oligopeptidic compound for use according to claim 1, wherein said oligopeptidic compound comprises or consists of the amino acid sequence set forth in SEQ ID NO: 1.

3. The oligopeptidic compound for use according to claim 1 or 2, wherein said oligopeptidic compound is an inverso-compound, every amino acid of which is a D-amino acid.

4. The oligopeptidic compound for use according to any one of claims 1 to 3, wherein said oligopeptidic compound is selectively cytotoxic towards cancer cells.

5. The oligopeptidic compound for use according to any one of claims 1 to 4, wherein the antibody is nivolumab, pembrolizumab, atezolizumab, durvalumab, tislelizumab or avelumab.

6. The oligopeptidic compound for use according to any one of claims 1 to 5, wherein said treatment comprises separately, simultaneously or sequentially administering said oligopeptidic compound and said antibody to said subject.

7. The oligopeptidic compound for use according to any one of claims 1 to 6, wherein said subject is a human.

8. The oligopeptidic compound for use according to any one of claims 1 to 7, wherein said neoplastic condition is cancer.

9. The oligopeptidic compound for use according to claim 8, wherein said cancer is cervical cancer, anal cancer, vaginal cancer, vulvar cancer, penile cancer, melanoma, lung cancer, a head and neck cancer, bladder cancer, kidney cancer, Hodgkin's lymphoma, a squamous cell carcinoma or Merkel cell carcinoma.

10. The oligopeptidic compound for use according to claim 8 or 9, wherein said cancer is microsatellite instability-high or mismatch-repair deficient.

11. The oligopeptidic compound for use according to any one of claims 8 to 10, wherein said subject is HPV-positive.

12. A kit for use according to claim 1 comprising an oligopeptidic compound as defined in claim 1 and an antibody as defined in claim 1.

13. The kit for use according to claim 12, wherein said oligopeptidic compound is as defined in any one of claims 2 to 4 and/or said antibody is as defined in claim 5.

14. A product comprising an oligopeptidic compound as defined in claim 1 and an antibody as defined in claim 1 for separate, simultaneous or sequential use in the treatment of a neoplastic condition in a subject.

15. The product for use of claim 14, wherein the oligopeptidic compound, antibody, neoplastic condition, treatment and/or subject are as defined in any one of claims 2 to 11.

## Patentansprüche

1. Oligopeptidische Verbindung, die eine Aminosäuresequenz, die in SEQ ID NR: 1 angegeben ist, oder eine Aminosäuresequenz mit mindestens 85 % Sequenzidentität mit dieser umfasst, zur Verwendung in der Behandlung einer neoplastischen Störung,
wobei die oligopeptidische Verbindung Aktivität zum Hemmen des Wachstums und/oder der Lebensfähigkeit neoplastischer Zellen aufweist,
und die Behandlung Verabreichen der oligopeptidischen Verbindung in Kombination mit einem Antikörper, der PD-1 oder PD-L1 bindet und die Interaktion zwischen PD-1 und PD-L1 blockiert, an ein Subjekt umfasst.

2. Oligopeptidische Verbindung zur Verwendung nach Anspruch 1, wobei die oligopeptidische Verbindung die Aminosäuresequenz, die in SEQ ID NR: 1 angegeben ist, umfasst oder aus dieser besteht.

3. Oligopeptidische Verbindung zur Verwendung nach Anspruch 1 oder 2, wobei die oligopeptidische Verbindung eine Inverso-Verbindung ist, von welcher jede Aminosäure eine D-Aminosäure ist.

4. Oligopeptidische Verbindung zur Verwendung nach einem der Ansprüche 1 bis 3, wobei die oligopeptidische Verbindung gegenüber Krebszellen selektiv zytotoxisch ist.

5. Oligopeptidische Verbindung zur Verwendung nach einem der Ansprüche 1 bis 4, wobei der Antikörper Nivolumab, Pembrolizumab, Atezolizumab, Durvalumab, Tislelizumab oder Avelumab ist.

6. Oligopeptidische Verbindung zur Verwendung nach einem der Ansprüche 1 bis 5, wobei die Behandlung separates, gleichzeitiges oder aufeinanderfolgendes Verabreichen der oligopeptidischen Verbindung und des Antikörpers an das Subjekt umfasst.

7. Oligopeptidische Verbindung zur Verwendung nach einem der Ansprüche 1 bis 6, wobei das Subjekt ein Mensch ist.

8. Oligopeptidische Verbindung zur Verwendung nach einem der Ansprüche 1 bis 7, wobei die neoplastische Störung Krebs ist.

9. Oligopeptidische Verbindung zur Verwendung nach Anspruch 8, wobei der Krebs Gebärmutterhalskrebs, Analkarzinom, Scheidenkrebs, Vulvakarzinom, Peniskarzinom, Melanom, Lungenkrebs, ein Kopf- und Halskrebs, Blasenkrebs, Nierenkrebs, Hodgkin-Lymphom, ein Plattenepithelkarzinom oder Merkelzellkarzinom ist.

10. Oligopeptidische Verbindung zur Verwendung nach Anspruch 8 oder 9, wobei der Krebs hoch an Mikrosatelliteninstabilität oder Mismatch-Repair-defizient ist.

11. Oligopeptidische Verbindung zur Verwendung nach einem der Ansprüche 8 bis 10, wobei das Subjekt HPV-positiv ist.

12. Kit zur Verwendung nach Anspruch 1, das eine oligopeptidische Verbindung wie in Anspruch 1 definiert und einen Antikörper wie in Anspruch 1 definiert umfasst.

13. Kit zur Verwendung nach Anspruch 12, wobei die oligopeptidische Verbindung wie in einem von Ansprüchen 2 bis 4 definiert ist und/oder der Antikörper wie in Anspruch 5 definiert ist.

14. Produkt, das eine oligopeptidische Verbindung wie in Anspruch 1 definiert und einen Antikörper wie in Anspruch 1 definiert zur separaten, gleichzeitigen oder aufeinanderfolgenden Verwendung in der Behandlung einer neoplastischen Störung bei einem Subjekt umfasst.

15. Produkt zur Verwendung nach Anspruch 14, wobei die oligopeptidische Verbindung, der Antikörper, die neoplastische Störung, die Behandlung und/oder das Subjekt wie in einem der Ansprüche 2 bis 11 definiert sind.

## Revendications

1. Composé oligopeptidique comprenant la séquence d'acides aminés représentée dans la SEQ ID NO:1, ou une séquence d'acides aminés présentant au moins 85 % d'identité de séquence à celui-ci, destiné à être utilisé dans le traitement d'une maladie néoplasique,
dans lequel ledit composé oligopeptidique présente une activité inhibitrice de la croissance et/ou de la viabilité de cellules néoplasiques,
et ledit traitement comprend l'administration à un sujet dudit composé oligopeptidique en association avec un anticorps qui se lie à PD-1 ou PD-L1 et bloque l'interaction entre PD-1 et PD-L1.

2. Composé oligopeptidique destiné à être utilisé selon la revendication 1, dans lequel ledit composé oligopeptidique comprend ou se compose de la séquence d'acides aminés représentée par la SEQ ID NO:1.

3. Composé oligopeptidique destiné à être utilisé selon la revendication 1 ou 2, dans lequel ledit composé oligopeptidique est un composé inverso, dont chaque acide aminé est un acide aminé D.

4. Composé oligopeptidique destiné à être utilisé selon l'une quelconque des revendications 1 à 3, dans lequel ledit composé oligopeptidique est sélectivement cytotoxique à l'égard des cellules cancéreuses.

5. Composé oligopeptidique destiné à être utilisé selon l'une quelconque des revendications 1 à 4, dans lequel l'anticorps est le nivolumab, le pembrolizumab, l'atézolizumab, le durvalumab, le tislelizumab ou l'avélumab.

6. Composé oligopeptidique destiné à être utilisé selon l'une quelconque des revendications 1 à 5, dans lequel ledit traitement comprend l'administration séparée, simultanée ou séquentielle dudit composé oligopeptidique et dudit anticorps audit sujet.

7. Composé oligopeptidique destiné à être utilisé selon l'une quelconque des revendications 1 à 6, dans lequel ledit sujet est un être humain.

8. Composé oligopeptidique destiné à être utilisé selon l'une quelconque des revendications 1 à 7, dans lequel la maladie néoplasique est un cancer.

9. Composé oligopeptidique destiné à être utilisé selon la revendication 8, dans lequel ledit cancer est un cancer du col de l'utérus, un cancer de l'anus, un cancer du vagin, un cancer de la vulve, un cancer du pénis, un mélanome, un cancer du poumon, un cancer de la tête et du cou, un cancer de la vessie, un cancer du rein, un lymphome de Hodgkin, un carcinome épidermoïde ou un carcinome à cellules de Merkel.

10. Composé oligopeptidique destiné à être utilisé selon la revendication 8 ou 9, dans lequel ledit cancer présente une forte instabilité des microsatellites ou une déficience dans la réparation des mésappariements.

11. Composé oligopeptidique destiné à être utilisé selon l'une quelconque des revendications 8 à 10, dans lequel ledit sujet est positif au VPH.

12. Kit destiné à être utilisé selon la revendication 1 comprenant un composé oligopeptidique tel que défini selon la revendication 1 et un anticorps tel que défini selon la revendication 1.

13. Kit destiné à être utilisé selon la revendication 12, dans lequel ledit composé oligopeptidique est tel que défini selon l'une quelconque des revendications 2 à 4 et/ou ledit anticorps est tel que défini selon la revendication 5.

14. Produit comprenant un composé oligopeptidique tel que défini selon la revendication 1 et un anticorps tel que défini selon la revendication 1 pour une utilisation séparée, simultanée ou séquentielle dans le traitement d'une maladie néoplasique chez un sujet.

15. Produit destiné à être utilisé selon la revendication 14, dans lequel le composé oligopeptidique, l'anticorps, la maladie néoplasique, le traitement et/ou le sujet sont tels que définis selon l'une quelconque des revendications 2 à 11.
